# EUROPEAN PATENT APPLICATION

(11) **EP 0 933 116 A1**
(43) Date of publication of application: **04.08.1999**
(21) Application number: 99300699.8
(22) Date of filing: 29.01.1999
(51) Int. Cl.: B01D 47/06, B01D 53/14

(54) **Means for removing odor produced by livestock**

(30) Priority: 02.02.1998 US 17234
(71) Applicant: Kim, Hee Jung, Seoul 138-220 (KR); Shin, Myoung Soo, Sungbuk-ku, Seoul 136-020 (KR)
(72) Inventor: Kim, Hee Jung, Songpa-ku, Seoul 138-220 (KR); Shin, Myoung Soo, Sungbuk-ku, Seoul 136-020 (KR); Park, Sang Chul, Seocho-ku, Seoul 137-044 (KR)
(74) Representative: Evens, Paul Jonathan

(57) **Abstract**

A means for removing odor emitting particles and molecules from the air comprising the circulation of air through a reactor and the spraying of the air with a highly polar water. The water is prepared through electrolysis and irradiation by far infrared light. The resulting water is highly polar and is efficient in removing odor emitting particles and molecules from the air. When the water is sprayed into the air, the odor emitting particles and molecules are attracted to the highly polar water droplets and adhere to the droplets. The water then settles and is flushed from the reactor . The water may be purged and recycled to the spray.

## Description

### BACKGROUND OF THE INVENTION

### 1. Field of the Invention

The present invention generally pertains to the removal of odors produced by livestock, and particularly to a process which utilizes mechanical equipment in order to remove odors from the particular area.

### 2. Description of the Prior Art

It is commonly known that unpleasant odors invariably and incessantly emanate from livestock farms. While this may be acceptable for the employees on the farm, odors tend to diffuse through the air, spreading to adjacent areas. Governmental ordinances regulate the amount/intensity of odor emanating from such odor-producing places, and thereby indirectly affect the amount of livestock such places may have, since the amount of livestock is proportional to amount of odor emanating from the place where the livestock is kept. Therefore, there is a need for a means in which odors may be effectively removed from an area.

Consequently, the primary purpose of the present invention is to provide a means in which odor may be removed from a geographical area.

### SUMMARY OF INVENTION

The present invention comprises the propagation of air by an air circulation system through a chemical spray, wherein odor emitting particles and molecules within the air passing through the spray are effectively purged from the air, thereby resulting in a relatively odor-free air. The particles and molecules are purged from the air through usage of electrolyzed water irradiated by far infrared light. Such water is notably more polar than normal water due to the dissociation of hydrogen bonds between water molecules and has a greater ability to strip such odor emitting particles and molecules from the air.

These together with other objects of the present invention are explained clearly in the claims annexed to and forming a part of this disclosure. For a better understanding of the invention, its operating advantages and the specific objects attained by its use, reference should be made to the accompanying drawings and descriptive matter in which there are illustrated preferred embodiments of the invention.

### BRIEF DESCRIPTION OF THE DRAWINGS

For a better understanding of the principle and nature of the present invention, reference should be made to the following detailed description taken in connection with the accompanying drawings in which;

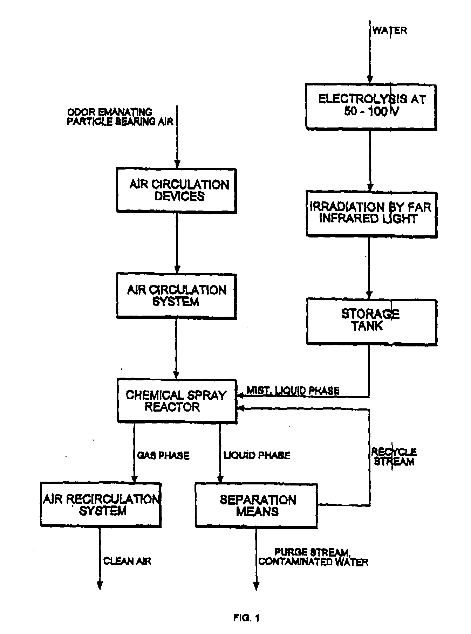

**FIG. 1** is a block flow diagram depicting the circulation system and chemical spray, and the manner in which the water used in the chemical spray is prepared.

### DETAILED DESCRIPTION OF THE PREFERRED EMBODIMENT

Referring to **FIG. 1,** the present invention comprises the circulation of air through a chemical spray, wherein the chemical used in the spray is water that has been electrolyzed and irradiated by far infrared light.

An electric current is passed through filtered distilled water, preferably within the range of 50-100 volts. The water is then irradiated by far infrared light, wherein the wavelength may vary depending upon the specific equipment used and the odors to be removed. The electrolysis of water dissociates water molecule clusters formed due to hydrogen bonding between water molecules, thereby resulting in smaller water molecule clusters with higher polarity because of a greater amount of random charge within each cluster.

Fans are used to create a flow of air through a plug now reactor, wherein a spraying means emits the prepared water as a fine mist into the rbactor. Because of the high polarity of the water odor emitting particles and molecules adhere to the fine water droplets, and the resulting contaminated water is collected. The contaminated water may be fed into a separating means, thereby regenerating the water, and the water may be recycled to the chemical spray.

## Claims

1. A process for removing odour emitting particles and molecules from air comprising:
a) inducing air circulation through a reactor;
b) spraying said air with a fine mist of water.

2. A process for removing odour emitting particles and molecules from air according to claim 1, wherein said water is electrolyzed and irradiated by far infrared light.

3. A process for removing odour emitting particles and molecules from air as mentioned in claim 1 or claim 2, wherein said water is cleaned and sprayed again to said air.

4. A process for removing odour emitting particles and molecules from air as mentioned in claim 2, wherein said water is electrolyzed with a current in the range of 50 to 100 volts.

5. A process for removing odour emitting particles and molecules from air according to any one of claims 2 to 4, wherein the electrolysed and irradiated water has smaller clusters of water molecules of higher polarity than tap water.

6. Apparatus for removing odour emitting particles and molecules from air, comprising a reactor chamber, means for inducing air with an odour to circulate through the reactor chamber, and means for spraying circulating air with a fine mist of water to remove said odour.

7. Apparatus according to claim 6, further comprising means for electrolysing water, means for irradiating with far infrared light the electrolysed water, and means for supplying electrolysed and irradiated water to the spraying means.
